# EUROPEAN PATENT SPECIFICATION

(11) **EP 3 968 992 B1**
(45) Date of publication and mention of the grant of the patent: **19.08.2026**
(21) Application number: 20806830.4
(22) Date of filing: 12.05.2020
(51) Int. Cl.: A61K 35/51, A61K 35/28, A61J 1/10, A61K 9/00, A61K 47/02, A61K 47/26, A61K 35/50, A61K 35/545, A61K 31/439, A61K 31/4545

(54) **PACKAGING AND SHIPPING OF HIGH-CONCENTRATION UMBILICAL CORD BLOOD CELLS**
VERPACKUNG UND VERSAND VON HOCHKONZENTRIERTEN NABELSCHNURBLUTZELLEN
CONDITIONNEMENT ET EXPÉDITION DE CELLULES SANGUINES DU CORDON OMBILICAL À UNE CONCENTRATION ÉLEVÉE

(30) Priority: 15.05.2019 US 201962848230 P
(43) Date of publication of application: 23.03.2022
(73) Proprietor: Stemcyte, Inc., Baldwin Park CA 91706 (US); Rutgers, the State University of New Jersey, New Brunswick, NJ 08901 (US)
(72) Inventor: CHEN, Monica, Baldwin Park, CA 91706 (US); TONAI, Richard, Baldwin Park, CA 91706 (US); YOUNG, Wise, New Brunswick, NJ 08901 (US); SUN, Dongming, Princeton Junction, NJ 08550 (US); WANG, Jonas, Baldwin Park, CA 91706 (US)
(74) Representative: Cabinet Becker et Associés
(86) International application number: PCT/US2020/032433
(87) International publication number: WO 2020/231968

(56) References cited:
- WO-A1-2012/151266
- WO-A1-2017/181110
- WO-A2-2013/126590
- CN-A- 106 922 648
- US-A- 5 955 257
- US-A1- 2008 286 324
- US-A1- 2010 068 191
- US-A1- 2018 271 911
- BAXTER: "PLASMA-LYTE A Injection pH 7.4 (Multiple Electrolytes Injection, Type 1, USP) in VIAFLEX Plastic Container", 30 September 2018 (2018-09-30), XP093045804, Retrieved from the Internet <URL:https://web.archive.org/web/20180830091221if_/http://baxterpi.com/pi-pdf/PlasmaLyte+A_Injection_+Viaflex_+PI.pdf> [retrieved on 20230510]
- BAXTER: "PLASMA-LYTE A Injection pH 7.4 (Multiple Electrolytes Injection, Type 1, USP) in VIAFLEX Plastic Container", 30 September 2018 (2018-09-30), XP93045804, Retrieved from the Internet <URL:https://web.archive.org/web/20180830091221if_/http://baxterpi.com/pi-pdf/PlasmaLyte+A_Injection_+Viaflex_+PI.pdf>

## Description

### FIELD OF THE INVENTION

This invention relates to processes and products for packaging and shipping therapeutic cells for cell therapy.

### BACKGROUND OF THE INVENTION

Cell therapy, which involve administering cells, such as stem or pluripotent cells, to a patient, have been used for treating various conditions. Stem or pluripotent cells are types of cells characterized by the ability to renew themselves through mitotic cell division and differentiate into a diverse range of specialized cell types. Accordingly, such cells have the potential to be used in treating a wide variety of diseases and injuries, including nervous system trauma, malignancies, genetic diseases, hemoglobinopathies, and immunodeficiency.

However, it has been shown that a loss of functional progenitors will occur if stem cells (such as placenta or cord blood cells) is not treated quickly after removal for freezing (Ivanovic et al., Transfusion, 2011 Sep;51(9):2044-5). Accordingly, applications of these cells are often hampered by logistical issues. For example, stem cells (such as cord blood cells), once collected, are routinely cryopreserved at storage facilities (such as cell banks) and, when needed, transported from the facilities to hospitals. This cryopreservation process, where cells or tissues are preserved by cooling to low sub-zero temperatures, typically 77 K or -196 °C (the boiling point of liquid nitrogen), entails certain risks. For example, cells preserved can be damaged due to freezing during the approach to low temperatures or warming to room temperature. These risks are particularly serious for stem or pluripotent cells as one of the most important aspects in such cell transplantation is the number of viable stem/pluripotent cells and their developmental potentials at time of transplantation. Out of this concern, stem/pluripotent cells are routinely shipped cryopreserved over a period as short as possible. Indeed, overnight shipments on dry ice or in a liquid nitrogen shipper are the industry standard and extra care must be taken to monitor the temperatures. Yet, this practice does not eliminate the risks.

Furthermore, for cryopreservation, it is a standard protocol to mix cells and a cryoprotectant to a final concentration of viable cells in the range between 10⁶ and 10⁷ cells per mL since cells frozen in lower or higher cell concentration often tend to have less viability (Kielberg et al., Cryopreservation of Mammalian Cells - Protocols, Tech Note No. 14, 2010 Thermo Fisher Scientific Inc., https:/ /assets.thermofisher.com/TFS-Assets/LSG/ Application-Notes/D19575.pdf). Yet, therapeutic applications of these cells often entail larger quantities and concentrations higher than 10⁷ cells/ml. As a result, clinicians have to further process these cells to increase the concentrations, thereby creating additional logistical and compliance issues.

Accordingly, it is extraordinarily costly and not practical for long-distance (*e.g*., transcontinental) transportation of stem cells. There is a need for processes or methods of shipping stem cells at high concentration and room/ambient temperatures

WO 2012/151266 A1 discloses a composition comprising 1x10⁷ immune cells/ml (suitable for therapeutic administration) in PlasmaLyte A buffer with 1% HSA. This document also discloses a method of storing cells in the same composition within syringes placed at a temperature of 4°C for 48 hours.

US 5 955 257 A discloses a composition comprising 1x10⁶ stem cells/ml suspended in PlasmaLyte-A, 1% HSA and 50 mM histidine, and stored in polymer bags at room temperature or at 4°C for 0-72 h. WO 2013/126590 A2 discloses a composition comprising CD34+ cells suspended in Plasma-Lyte A with 5% HSA to a concentration of 5 or 7.5x10⁶ cells/ml, loaded into polycarbonate syringes (which in one examples were packed into thermal shipping containers) and stored at 2-8°C or 1-10°C for 0-3 days.

### SUMMARY OF INVENTION

This invention addresses the need mentioned above in a number of aspects.

The invention is as defined in the appended claims.

In one aspect, the invention provides a therapeutic composition comprising (i) about 1 x 10⁷ to 1 x 10⁹ /ml (*e.g.,* 2 x 10⁷ to 1 x 10⁹/ml, 5 x 10⁷ to 1 x 10⁹/ml, 1 x 10⁸/ml, 2 x 10⁸/ml, 3 x 10⁸/ml, 4 x 10⁸/ml, 5 x 10⁸/ml) therapeutic cells that are umbilical cord blood cells and (ii) a pharmaceutically acceptable carrier solution. The pharmaceutically acceptable carrier solution (a) contains about 25-30 mM (*e.g.,* 26-28 mM and 27 mM) acetate and about 20-25 mM (*e.g.,* 21-24 mM and 23 mM) gluconate and (b) has an osmolality of about 270 to 320 mOsmol/L (*e.g.,* 280-310, 280-300, 290-300, and about 294 or 295 mOsmol/L). The therapeutic composition is free of DMSO or contains a trace amount of DMSO (*i.e.,* 0.5% or lower.). The pharmaceutically acceptable carrier solution can have 126-154 mEq/L sodium.In some embodiments, the pharmaceutically acceptable carrier solution can contain one or more of the following: about 120-160 mM (*e.g.,* 130-150 and 140 mM) Na⁺, about 3-7 mM (*e.g.,* 4-6 and 5 mM) K⁺, about 1.0-2.0 mM (*e.g.,* 1.2-1.8 and 1.5 mM) Mg²⁺, and about 90-110 (*e.g.,* 95 - 100 and 98 mM) mM Cl⁻. In some embodiments, the pharmaceutically acceptable carrier solution is free of Ca²⁺, or lactate or both.

In one example, the pharmaceutically acceptable carrier solution contains: about 140 mM Na⁺, about 5 mM K⁺, about 1.5 mM Mg²⁺, about 98 mM Cl⁻, about 27 mM acetate, and about 23 mM gluconate. In that case, the pharmaceutically acceptable carrier solution can contain about 90 mM Sodium Chloride (NaCl), about 5 mM Potassium Chloride (KCl), about 1.5 mM Magnesium Chloride (MgCl₂•6H₂O), about 27 mM Sodium Acetate Trihydrate (C₂H₃NaO₂•3H₂O), and about 23 mM Sodium Gluconate (C₆H₁₁NaO₇).

The therapeutic composition or the pharmaceutically acceptable carrier solution can have a pH of about 4.0 to 8.0 (*e.g.,* about 5.5 to about 8.0, about 6.0 to about 7.5, about 6.0, and about 7.4). The therapeutic composition is free of DMSO or contains a trace amount of DMSO (*i.e.,* 0.5% or lower.). The above-described therapeutic composition can contain about 0.5% to about 5% (*e.g.,* about 1% to about 5%, about 1-3%, about 1-2.5%, or about 1%) serum or serum albumin. Examples include human serum or human serum albumin (HSA). The therapeutic composition can have a temperature within the range of about 1 - 10 °C, about 2 - 8 °C, or about 3 - 5 °C. Preferably, the composition has a temperature of about 4 °C.

According to the invention, the therapeutic cells are umbilical cord blood cells. In some embodiments, the therapeutic composition or cells comprise CD13⁺, CD34⁺, or CD134⁺ cells. In one embodiment, the above-mentioned cells can be those that have been frozen and thawed, *e.g.,* those obtained from a blood bank. In that case, the composition can contain DNAse (*e.g.,* human DNAse) of about 10-100 U/ml, *e.g.,* 10, 15, 20, 25, 30, 40, 50, 60, 70, 80, 90, or 100 U/ml. Alternatively, the cells can be freshly obtained from a donor and have not been frozen and, in this case, DNAse is not necessary and the composition can be free of DNAse.

In a second aspect, the invention features a packaging product that contains a composition described above in a container comprising a substrate; the substrate has a polymer. In some examples, the polymer can be polytetrafluoroethylene (PTFE), perfluoroalkoxy (PFA), fluorinated ethylene propylene (FEP), polyvinylidene fluoride (PVDF), polyethylene, or polyvinyl chloride (PVC), which has properties of low friction or non-stickiness. The polymer can also be other polymers suitable for biologicals, such as ultra-low density polyethylene, low-density polyethylene (LDPE), linear low density polyethylene (LLDPE), high density polyethylene (HDPE), coaxially oriented polypropylene (COPP), biaxially oriented polypropylene (BOPP), polyethylene terephthalate (PET), polymide resins such as nylon, ethylene vinyl alcohol polymer (EVOH), and their metalized versions.

The packaging product can be in any suitable shapes, including, but not limited to, a bag, a syringe or a vial for an injector. In one example, the product is pre-filled with pluripotent cells for clinical uses. The pluripotent cells can be stem cells, such as hematopoietic stem cells or mesenchymal stem cells. The composition contains cord blood cells.

The present disclosure features a method for making the above-mentioned packaging product. The method includes steps of (a) providing a composition containing cells (such as pluripotent cells); (b) providing a container comprising a substrate, wherein the substrate comprises a polymer; (c) placing the composition in the container; and, (d) sealing the container.

In a third aspect, the invention provides a method for storing or shipping therapeutic cells which are umbilical cord blood cells, such as pluripotent or mononuclear cells. The method comprises (i) providing the therapeutic composition described above and (ii) storing or shipping the composition for about 24 to 96 hours, such as about 24 to about 72 hours at a temperature within the range of 1 - 10 °C. The above-mentioned packaging product may then be provided and delivered to a recipient, such as a courier, an agent or personnel of a receiving hospital.

During the delivering step, the temperature can be within the range of 1-10 °C, such as 1-7 °C, 2-6 °C, 3-5 °C, or about 4 °C. Using the method, the cells can be delivered over 12-96 hours, *e.g.,* at least 24, 36, 38, 60, 72, 84, or 96 hours. In one example, the therapeutic composition is stored or shipped for about 72 hours.

Upon the delivering, the pluripotent or mononuclear cells can have a total nucleated cells (TNC) recovery rate of more than 40% (*e.g.,* 45%, 50%, 55%, 60%, 65%, 70%, 75%, 80%, 85%, 90%, and 95%). In addition, upon the delivering, the pluripotent cells can have a viability (as determined by the AO/PI method disclosed herein) of more than 60% (*e.g.,* 65%, 70%, 75%, 80%, 85%, 90%, and 95%).

In an embodiment, upon the delivering, the pluripotent or mononuclear cells can have at least 0.25% CD34⁺CD45⁺ cells (*e.g.,* 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.1%, 1.15%, 1.2%, 1.3%, 1.4%, 1.5.%, 1.6%, 1.7%, 1.8%, 1.9.%, or 2.0 %).

In another embodiment, upon the delivering, the pluripotent or mononuclear cells can have at least 0.10% CD133⁺ cells (*e.g.,* 0.10%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.1%, 1.15%, 1.2%, 1.3%, 1.4%, 1.5.%, 1.6%, 1.7%, 1.8%, 1.9.%, or 2.0 %).

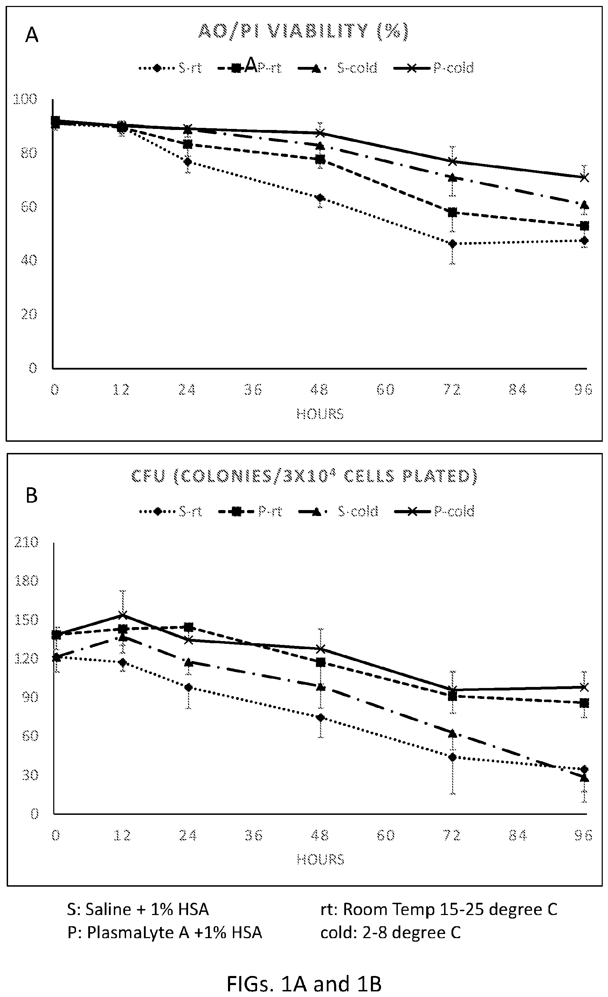

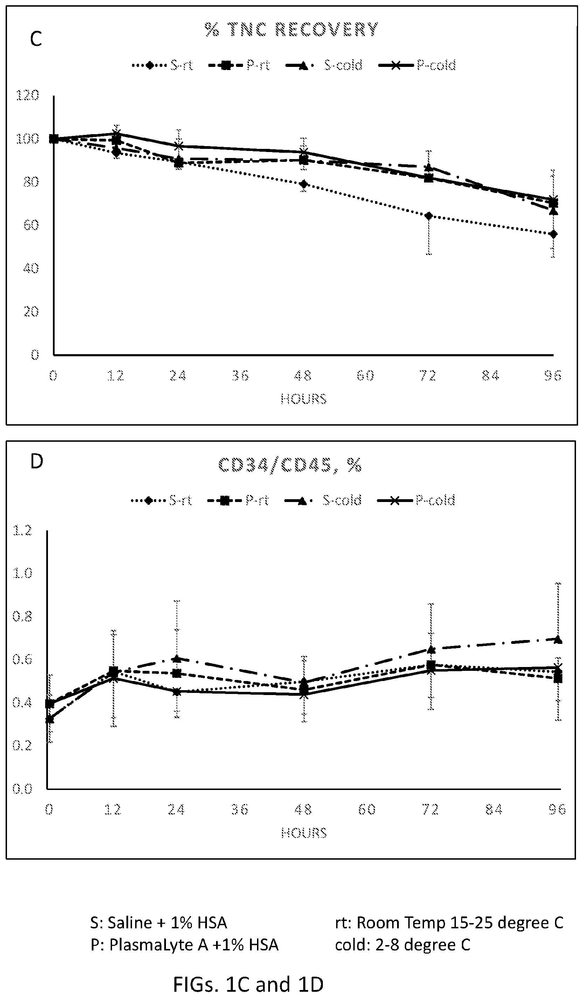

In yet another embodiment, upon the delivering, the pluripotent or mononuclear cells are capable of forming a large number of colony forming unit (CFU) colonies per plate. For example, as shown in FIG. 1, the cells are capable of forming at least 30 (*e.g.,* 40, 50, 60, 70, 80, 90, 95, 100, or 110) CFU colonies per 3x10⁴ cells plated after being stored or shipped at about 2 to about 8°C over 72 hours.

The above-mentioned values can be determined according to the methods known in the art or described in the examples below.

The details of one or more embodiments of the invention are set forth in the description below.

### BRIEF DESCRIPTION OF THE DRAWINGS

FIGs. 1A, 1B, 1C, and 1D are a set of diagrams showing results of stability study of cells after storing or shipping in saline or a composition of this invention at room temperature or 4 °C.

### DETAILED DESCRIPTION OF THE INVENTION

The present invention relates to packaging and/or shipping stem/pluripotent cells (i.e. , umbilical cord blood) or preparations containing such cells under conditions such as room or ambient temperature (*i.e.,* 1-25 °C) at a high concentration over an extended period (*e.g.,* 12 to 72 hours). Stem/pluripotent cells and preparations thus packaged and shipped unexpectedly had satisfactory viabilities and development potentials for clinical uses.

As mentioned above, it was known in the art that a loss of functional progenitors will occur if stem cells (such as placenta or cord blood cells) are not treated quickly after removal for freezing (Ivanovic et al., Transfusion, 2011 Sep;51(9):2044-5). Yet, in many cases, particularly when the location (*e.g.,* a hospital or a maternity clinic) where cord blood cells are collected, expanded, concentrated, or processed is a long way from the location at which the cells are used to treat patients, it is difficult to perform the treatment within 24 hours. In addition, regardless of the manufacturing process, therapeutic cell compositions need to satisfy strict regulatory guidelines. For example, a therapeutic cell composition should have sufficiently high cell concentration (*e.g*., 1 x 10⁷/ml, 1 x 10⁸/ml). To that end, conventional stem/pluripotent cells packaging and shipping do not allow one to ship cells at such high cell concentrations. To obtain therapeutic composition with such high cell concentrations, clinicians have to further process the cells to increase the cell numbers and/or concentrations.

However, since expansion or concentration of cells is considered to be more than minimal manipulation, cells that are expanded or concentrated are more strictly regulated than those that are simply obtained from a donor and given to a recipient with only minimal manipulation. In the U.S., therapeutic cells must be manufactured in a manner consistent with Current Good Manufacturing Practice (cGMP) regulations enforced by the US Food and Drug Administration (FDA). As treatment centers may or may not be cGMP compliant, conventional stem/pluripotent cells packaging and shipping limit applications of these cells.

The invention disclosed herein allows one to preserve and ship stem/pluripotent cells from umbilical cord blood at sufficiently high concentrations over an extended period under ambient temperatures, while maintaining viability and functionality of at least the stem cells and hematopoietic progenitors. Accordingly, the invention addresses the need for processes or methods of shipping umbilical cord blood stem cells at high concentration and under room/ambient temperatures

### Therapeutic Compositions

One aspect of the invention relates to a therapeutic cell composition. The composition comprises (i) about 1 x 10⁷ to 1 x 10⁹/ml therapeutic cells that are umbilical cord blood cells and (ii) a pharmaceutically acceptable carrier solution. The pharmaceutically acceptable carrier solution (a) contains about 25-30 mM (*e.g.,* 26-28 mM and 27 mM) acetate and about 20-25 mM (*e.g.,* 21-24 mM and 23 mM) gluconate and (b) has an osmolality of about 270 to 320 mOsmol/L (*e.g.,* 280-310, 290-300 and about 294 or 295 mOsmol/L).

The term "stem cell" refers to any cell that is capable of differentiating into a number of final, differentiated, specialized cell types. Stem cells emanate from all germinal layers (*i.e.,* ectoderm, mesoderm, and endoderm). Typical sources of stem cells include bone marrow, peripheral blood, umbilical cord blood, placental blood, muscle tissue, and adipose tissue.

Stem cells may be totipotent or pluripotent. Totipotent stem cells typically have the capacity to develop into any cell type. Totipotent stem cells can be both embryonic and non-embryonic in origin. Pluripotent cells are typically cells capable of differentiating into several different, final differentiated cell types. For example, pluripotent stem cells can give rise to cells of the nervous system, skin, liver, kidney, blood, muscle, bone, etc. Examples of pluripotent stem cells include, but are not limited to, cord blood stem cells, neural stem cells, hematopoietic stem cells, adipose-derived stem cells, mesenchymal stem cells, placental-derived stem cells, exfoliated tooth-derived stem cells, and hair follicle stem cells. In contrast, multipotent or adult stem cells typically give rise to limited types of cells. The term stem cell as used herein includes progenitor cells unless otherwise noted. Unipotent stem cells can produce only one cell type, but have the property of self-renewal that distinguishes them from non-stem cells. These stem cells can originate from various tissue or organ systems, including, but not limited to, blood, nerve, muscle, skin, gut, bone, kidney, liver, pancreas, thymus, and the like. In accordance with the present invention, the stem cell is derived from umbilical cord blood.

The cells described in this invention can be substantially pure. The term "substantially pure", when used in reference to stem cells or cells derived therefrom (*e.g.*, differentiated cells), means that the specified cells constitute a substantial portion of or the majority of cells in the preparation (*i.e.,* more than 20%, 30%, 40%, 50%, 60%, 70%, 80%, 90%, or 95%). For example, a substantially purified population of cells constitutes at least about 70% of the cells in a preparation, usually about 80% of the cells in a preparation, and particularly at least about 90% of the cells in a preparation (*e.g.*, 95%, 97%, 99% or 100%).

According to the invention, umbilical cord blood cells are used. These cells can be obtained as described in the example section below or by methods known in the art and then tested by standard techniques. To confirm the differentiation potential of the cells, they can be induced to form, for example, various colony-forming units, by methods known in the art. The cells thus confirmed can be further propagated in a non-differentiating medium culture for more than 10, 20, 50, or 100 population doublings without indications of spontaneous differentiation, senescence, morphological changes, increased growth rate, or changes in ability to differentiate into neurons. The cells can be stored by standard methods before use.

### Hematopoietic Stem Cells

Hematopoietic stem cell is pluripotent and ultimately gives rise to all types of terminally differentiated blood cells. The hematopoietic stem cell can self-renew, or can differentiate into more committed progenitor cells, which progenitor cells are irreversibly determined to be ancestors of only a few types of blood cell. For instance, a hematopoietic stem cell can differentiate into (i) myeloid progenitor cells, which myeloid progenitor cells ultimately give rise to monocytes and macrophages, neutrophils, basophils, eosinophils, erythrocytes, megakaryocytes/platelets, dendritic cells, or (ii) lymphoid progenitor cells, which lymphoid progenitor cells ultimately give rise to T-cells, B-cells, and lymphocyte-like cells called natural killer cells (NK-cells). Once the stem cell differentiates into a myeloid progenitor cell, its progeny cannot give rise to cells of the lymphoid lineage, and, similarly, lymphoid progenitor cells cannot give rise to cells of the myeloid lineage. For a general discussion of hematopoiesis and hematopoietic stem cell differentiation, see Chapter 17, Differentiated Cells and the Maintenance of Tissues, Alberts et al., 1989, Molecular Biology of the Cell, 2nd Ed., Garland Publishing, New York, N.Y.; Chapter 2 of Regenerative Medicine, Department of Health and Human Services, August 2006, and Chapter 5 of Hematopoietic Stem Cells, 2009, Stem Cell Information, Department of Health and Human Services.

*In vitro* and *in vivo* assays have been developed to characterize hematopoietic stem cells, for example, the spleen colony forming (CFU-S) assay and reconstitution assays in immune-deficient mice. Further, presence or absence of cell surface protein markers defined by monoclonal antibody recognition have been used to recognize and isolate hematopoietic stem cells. Such markers include CD34, CD38, CD43, CD45RO, CD45RA, CD59, CD90, CD109, CD117, CD133, CD166, and HLA DR, and combinations thereof. See Chapter 2 of Regenerative Medicine, Department of Health and Human Services, August 2006 and the references cited therein.

### Cord Blood Cells

Human umbilical cord blood and/or human placental blood are sources of cord blood stem cells. Such blood can be obtained by any method known in the art. The use of cord or placental blood as a source of stem cells provides numerous advantages, including that the cord and placental blood can be obtained easily and without trauma to the donor. See, *e.g.,* U.S. Pat. No. 5,004,681 and U.S. Pat. No. 7,147,626. Collections should be made under sterile conditions. Immediately upon collection, cord or placental blood can be mixed with an anticoagulent. Such an anticoagulent can be any known in the art, including CPD (citrate-phosphate-dextrose), ACD (acid citrate-dextrose), Alsever's solution (Alsever et al., 1941, N.Y. St. J. Med. 41:126), De Gowin's Solution (De Gowin, et al., 1940, J. Am. Med. Ass. 114:850), Edglugate-Mg (Smith, et al., 1959, J. Thorac. Cardiovasc. Surg. 38:573), Rous-Turner Solution (Rous and Turner, 1916, J. Exp. Med. 23:219), other glucose mixtures, heparin, ethyl biscoumacetate, etc. See, *e.g.,* Hurn, 1968, Storage of Blood, Academic Press, New York, pp. 26-160). The cord blood can be obtained by direct drainage from the cord and/or by needle aspiration from the delivered placenta at the root and at distended veins. See, generally, U.S. Pat. No. 5,004,681.

In certain embodiments, the following tests on the collected blood sample can be performed either routinely, or where clinically indicated: (i) bacterial culture to ensure the absence of microbial contamination, established assays can be performed, such as routine hospital cultures for bacteria under aerobic and anaerobic conditions; and (ii) diagnostic screening for pathogenic microorganisms to ensure the absence of specific pathogenic microorganisms, various diagnostic tests can be employed. Diagnostic screening for any of the numerous pathogens transmissible through blood can be done by standard procedures. As one example, the collected blood sample can be subjected to diagnostic screening for the presence of Human Immunodeficiency Virus-1 or 2 (HIV-1 or HIV-2) using any of numerous assay systems based on the detection of virions, viral-encoded proteins, HIV-specific nucleic acids, antibodies to HIV proteins, etc. The collected blood can also be tested for other infectious diseases, including human T-Cell lymphotropic virus I and II (HTLV-I and HTLV-II), Hepatitis B, Hepatitis C, Cytomegalovirus, Syphilis, West Nile Virus and other infectious agents as designated by relevant regulatory authorities such as the U.S. Food and Drug Administration.

Preferably, prior to collection of the cord blood, maternal health history is determined in order to identify risks that the cord blood cells might pose in transmitting genetic or infectious diseases, such as cancer, leukemia, immune disorders, neurological disorders, hepatitis or AIDS. The collected cord blood samples can undergo testing for one or more of cell viability, HLA typing, ABO/Rh typing, CD34⁺ cell count, and total nucleated cell count.

Once the umbilical cord blood blood is collected from a single human at birth, the blood can be processed to produce an enriched hematopoietic stem cell population, or enriched hematopoietic stem and progenitor cell population, forming a population of cord blood stem cells. The hematopoietic stem cells, or hematopoietic stem and progenitor cells, can be positive for a specific marker expressed in increased levels on the hematopoietic stem cells or hematopoietic stem and progenitor cells, relative to other types of hematopoietic cells. For example, such markers can be CD34, CD43, CD45RO, CD45RA, CD59, CD90, CD109, CD117, CD133, CD166, HLA DR, or a combination thereof. The hematopoietic stem cells, or hematopoietic stem and progenitor cells, also can be negative for a specific marker, relative to other types of hematopoietic cells. For example, Lin is a combination of lineage-specific antibodies that serve as negative markers. CD38 also provides an example of a negative marker. Preferably, the hematopoietic stem cells, or hematopoietic stem and progenitor cells, are CD34+ cells.

Optionally, prior to enrichment for mononuclear cells (MNCs), hematopoietic stem cells or hematopoietic stem and progenitor cells, the red blood cells (RBCs) and white blood cells (WBCs) of the cord blood can be separated. Once the separation of the red blood cells and the white blood cells has taken place, the red blood cell fraction can be discarded, and the white blood cell fraction can be processed in the magnetic cell separator as above. Separation of the white and red blood cell fractions can be performed by any method known in the art, including centrifugation techniques. Other separation methods that can be used include the use of commercially available products FICOLL or FICOLL-PAQUE or PERCOLL (GE Healthcare, Piscataway, N.J.). FICOLL-PAQUE is normally placed at the bottom of a conical tube, and the whole blood is layered above. After being centrifuged, the following layers will be visible in the conical tube, from top to bottom: plasma and other constituents, a layer of MNCs called buffy coat containing the peripheral blood mononuclear cells (white blood cells), FICOLL-PAQUE, and erythrocytes and granulocytes, which should be present in pellet form. This separation technique allows easy harvest of the peripheral blood mononuclear cells.

Optionally, prior to CD34+ cell selection, an aliquot of the fresh cord blood unit can be checked for total nucleated cell count and/or CD34+ content. In particular embodiments, after the CD34+ cell selection, both CD34+ ("CB Stem Cells") and CD34-cell fractions are recovered. Optionally, DNA can be extracted from a sample of the CD34-cell fraction for initial HLA typing and future chimerism studies, even though HLA matching to the patient is not done. The CD34+ enriched stem cell fraction can be subsequently processed prior to expansion, for example, the stem cells can be suspended in an appropriate cell culture medium for transport or storage.

In particular embodiments, the umbilical cord blood blood sample are red cell depleted, and the number of CD34+ cells in the red cell depleted fraction is calculated.

### Pharmaceutically Acceptable Carriers

The therapeutic composition described herein includes a pharmaceutically acceptable carrier or preservation solution that contains about 25-30 mM (*e.g.,* 26-28 mM and 27 mM) acetate and about 20-25 mM (*e.g.,* 21-24 mM and 23 mM) gluconate and (b) has an osmolality of about 270 to 320 mOsmol/L (*e.g.*, about 280-310, 280-300, 290-300, 294, or 295 mOsmol/L).

In some embodiments, the carrier/preservation solution includes a solution of electrolytes or a cell or tissue preservation solution. In some particular embodiments, the carrier/preservation solution is not a cell growth culture medium. That is, the solution lacks one or more nutrients necessary for cell growth (such as a source of amino acids and nitrogen or a carbon source). For example, the preservation solution can comprise a solution of electrolytes only. The solution of electrolytes can comprise for example sodium, potassium, calcium, chloride, zinc, iron and/or magnesium ions.

Preferably, the pharmaceutically acceptable carrier or preservation solution is an isotonic, sterile, nonpyrogenic solution that contains no bacteriostatic or antimicrobial agents or added buffers. In that case, examples include physiologically balanced crystalloid solutions with multiple different formulations as long as they closely mimics human plasma in its content of electrolytes, osmolality, and pH. These solutions also have additional buffer capacity and contain anions such as acetate, gluconate, and even lactate that are converted to bicarbonate, CO₂, and water. Normal physiologic isotonicity range is approximately 280 - 310 mOsmol/liter. Such an electrolyte solution may for example be PLASMA-LYTE A or PLASMA-LYTE 148, which has an osmolarity of about 294 or 295 mOsmol/liter. PLASMA-LYTE A or PLASMA-LYTE 148 contains about 90 mM NaCl, about 5 mM KCl, about 1.5 mM MgCl₂, about 27 mM Sodium Acetate Trihydrate, and about 23 mM Sodium Gluconate. While PLASMA-LYTE A has a pH of about 7.4, PLASMA-LYTE 148 has a pH of about 6.0.

As a variant, the carrier/preservation solution may also comprise a buffer and/or one or several antioxidants. The buffer may for example be chosen from among physiological buffers (sulphate, phosphate or carbonate) or synthetic buffers (HEPES). Examples of antioxidants include free radical traps; iron chelators such as deferoxamine; vitamin E, vitamin C or sodium erythorbate; and thiolated derivatives such as N-acetylcysteine, glutathion or reduced glutathion.

The compositions disclosed herein allow one to preserve and ship stem/pluripotent cells from umbilical cord blood over an extended period under ambient temperatures, while maintaining viability and functionality of at least the stem cells and hematopoietic progenitors. In particular, compositions can have cell concentrations sufficiently high for clinical uses.

As disclosed herein, in some examples, after about 72 hours (3 days) of shipping or storage, the compositions can give a content of viable CD34+ hematopoietic stem cells equal to at least 80%, particularly at least 90% and even more particularly close to 100%, in relation to the number of viable CD34+ cells in the placental blood unit immediately after removal. After 3 days of storage/shipping, the storage/shipping method can give a content of viable hematopoietic progenitors equal to at least 75% and particularly at least 80% and even more particularly at least 90% in relation to the number of viable progenitors in the placental blood unit immediately after removal.

### Packaging Products

In another aspect, the present invention relates to packaging and/or shipping the therapeutic stem cells from umbilical cord blood or preparations described above under conditions such as room or ambient temperature over an extended period at a high concentration. The cells and preparations thus packaged and shipped unexpectedly had satisfactory viabilities and development potentials for clinical uses.

In one example, cord blood cells can be collected on site at a hospital or obtained from a cord blood bank (such as that maintained by STEMCYTE Inc.). Although any art-recognized procedures for collecting and storage can be used, a preferred procedure is described in the examples below and WO2012112572.

Generally, sterility test for various infectious markers should be conducted. In addition, total cell number, CD34+ cell number, and unit volume should be determined and recorded before be freezing for cryopreservation. The cryopreserved collected blood contains RBCs, which tend to break down during freezing and thawing. In that case, once lysed, DNA of RBCs increases viscosity of the collected cord blood cells and hinders further handling of the cord blood cells for clinical uses. To prevent this, DNAse can be added to the collected cells before cryopreservation for breaking down DNA. Doing so can reduce stickiness and clumping of cells and thereby, allow better separation of cells in osmotic gradients (*e.g*., FICOLL). A number of commercially available DNAses can be used. Examples include PULMOZYME^{®} marketed by GENENTECH.

Alternatively, the cord blood can be processed to remove red blood cells so that red blood cell is substantially depleted. If desired, the cord blood can be separated into a number of useful units (*e.g.,* total mononuclear cells (TMN), white blood cells, lymphocytes, CD34+ cells, CD133+ cells, macrophages, and other cells) by osmotic gradients (*e.g*., FICOLL) or in the manner described in Example 1 below. Also, as mentioned above, the cord blood cells to be shipped can be freshly obtained from a donor and have not been frozen. In these approaches, DNAses are not necessary during packaging and/or shipping such fresh units. Furthermore, plasma can be depleted according to methods known in the art, *e.g.,* those described in US Application 20080166324.

Then, the collected cells are packaged and prepared for shipping in a processing facility either on site in the hospital or off site at, *e.g.,* the above-mentioned blood bank. If the cells have been cryopreserved, they can be thawed in the manner described in WO2012112572. Again, sterility test for various infectious markers can be conducted and total cell numbers, CD34+ cell numbers, concentrations, and unit volume should be determined and recorded. Then, the cells are placed in the above-described container to form a package for shipping by a designated carrier.

As described above, while a number of pharmaceutically acceptable carrier or preservation solution can be used, isotonic or physiologically balanced salt solutions, such as Plasma-Lyte A, are preferred. These solutions preserve cells at a very high concentration and result in better cell survival rate. In addition, they are capable of maintaining long-term pH stability without atmospheric CO₂ (0.04%). The cells thus packaged and shipped can be administered as a pharmaceutical composition to a subject in need thereof directly without any further processing (*e.g*., further concentrating).

As disclosed herein, the material of the container can be any suitable material and preferably one approved for clinical uses. In general, the material can be a polymer that is of low friction or non-stickiness to cells, and not toxic to cells or harmful to stem cells recipients. Examples of suitable polymer include, but not limited to, polytetrafluoroethylene (PTFE), perfluoroalkoxy (PFA), fluorinated ethylene propylene (FEP), polyvinylidene fluoride (PVDF), polyethylene, polyvinyl chloride (PVC), ultra-low density polyethylene, low-density polyethylene (LDPE), linear low density polyethylene (LLDPE), high density polyethylene (HDPE), coaxially oriented polypropylene (COPP), biaxially oriented polypropylene (BOPP), polyethylene terephthalate (PET), polymide resins such as nylon, ethylene vinyl alcohol polymer (EVOH), and their metalized versions. Other polymers can also be used if their coefficients of frictions (against polished steel) are comparable to or lower than those of the above-mentioned polymer. Coefficients of frictions of the above-mentioned polymers are known in the art. For examples, coefficients of frictions can be lower than 0.5, such as 0.4, 0.3, 0.2, or 0.1. In preferred embodiment, one can use PTFE, PFA, PEP, or PVDF-based container marketed by DUPONT under the brand TEFLON, HYCLONE'S polyethylene-based containers, or TERUMO's PVC-based containers.

The substrate of the container can be formed into any shapes suitable for receiving and holding cells. Examples of the shapes include, but are not limited to, a bag, a tube, a syringe or a vial for an injector. In some embodiments, the substrate is formed in a shape suitable for culture or for a site of stem cell transplantation or implantation in various tissues, such as CNS. Examples include a tape, a membrane, a thread, a slide, a micro-bead, a micro-particle, a cell culture plate, a multi-well plate, and a bioreactor, all of which can receive cells.

As described herein, during the shipping, the cells in the package do not have to be kept at a lower temperature, *e.g.,* cryopreserved, or delivered overnight. Instead, the cells can be shipped within a rather broader temperature range, including room temperature, over a fairly extended period of time (*e.g.,* 1-8 days). Despite these less stringent conditions, it is preferred that the package is shipped in a temperature-protected container and/or monitored with a temperature probe to provide a shipper or recipient with the information if needed. Due to the less stringent conditions, the costs associated with shipping cryopreserved cells are avoided. In addition, as the shipping time can be as long as 1-8 days, long-distance, such as transcontinental, shipping becomes practical. As a result, patients who are far away from a source of particular stem cells (*e.g.,* those having a rare, matched HLA-type) will be able to benefit from stem cell transplantation.

Upon receipt of cells from a courier, the cells can be processed in the manner described in the examples below and tested for their suitability for transplantation. To this end, the following four criteria can be used to determine whether mononuclear cells shipped are suitable for transplantation.

### Cell Count

There must be enough viable cells for transplantation and analyses. Preferably, at least twice the number of cells needed for transplantation (*e.g*., into the spinal cord) are preferred so that there would be enough leftover cells for analyzing the cells. If the shipment contains fewer cells, the shipment should not be suitable for transplantation.

### Viability

Too many dead cells should be avoided in the preparation. To this end, manual count using Trypan Blue Exclusion (TBE) can be used as a criterion of viability. Expressed as a percentage, the TBE of the cell suspension represents non-blue-stained cells divided by the total number of stained and unstained cells. For cells designated for transplantation, TBE should be at least 70%. In general, wash procedures as described in the examples below eliminate dead cells and the cell suspensions typically have a TBE greater than 90% just before transplantation.

### Contamination

Any evidence or risk of contamination should be reported. This includes, for example, the presence of any leakage of fluids in the shipping bags, abnormal turbidity in the cell suspensions, bacteria or fungi visible under the microscope, or report of previous contamination. As disclosed herein, care should be taken to exclude cord blood units that are positive for maternal hepatitis B core antigen, as well as all other infectious agents that would normally exclude a cord blood unit from registration under National Marrow Donor Program (NMDP).

### Mononuclear cells

The final preparation should have 95% or more mononuclear cells. If the viability count of the cells reveals more than 5% other cells, such as red blood cells or neutrophils, the cells will not be used for transplantation. Note that there may be some immature red nucleated cells in umbilical cord blood.

In the above-described procedures, antibiotics can be added to a cell preparation. For example, gentamycin can be added at the beginning of cell processing to reduce risk of contamination during processing and shipping. The gentamycin may suppress bacterial growth even though multiple past media fill tests have dictated that contamination was not being introduced.

In the above described procedures, the cord blood stem cells can be further treated to expand the pool of stem cells, *i.e., in vitro* expansion, using methods such as those described in US Applications 20100189696, 20100323920, 20080227197, and 20080166324. The term *"in vitro* expansion" refers to the cultivation of stem cells in the laboratory. Such cells can be extracted from a mammal and additional quantities of cells generated by cultivation in the appropriate environment, *e.g.,* in media containing a lithium salt. If possible, stable cell lines are established to allow for continued propagation of cells.

### Uses

Embodiments of the present disclosure also relate to the commercial provision of the possibility to manufacture, store, or ship therapeutic cells under cGMP regulations enforced by the US FDA or the equivalent regulatory authority in non-US countries. The therapeutic cells and compositions are useful for treating a variety of diseases and disorders. Examples of the disorders include, but not limited to, a degenerative disease, ischemic conditions (*e.g*., limb ischemia, congestive heart failure, cardiac ischemia, kidney ischemia and ESRD, stroke, and ischemia of the eye), conditions requiring organ or tissue regeneration (*e.g*., regeneration of liver, pancreas, lung, salivary gland, blood vessel, bone, skin, cartilage, tendon, ligament, brain, hair, kidney, muscle, cardiac muscle, nerve, and limb), inflammatory diseases (*e.g*., heart disease, diabetes, spinal cord injury, rheumatoid arthritis, osteo-arthritis, inflammation due to hip replacement or revision, Crohn's disease, and graft versus host disease) autoimmune diseases (*e.g*., type 1 diabetes, psoriasis, systemic lupus, and multiple sclerosis), a congenital disease hematologic disorders such as anemia, neutropenia, thrombocytosis, myeloproliferative disorders or hematologic neoplasms and cancer such as leukemia and lymphoma.

### Definitions

As used herein, "therapeutic cells" refers to a cell population that ameliorates a condition, disease, and/or injury in a patient. Therapeutic cells may be autologous (*i.e.,* derived from the patient), allogeneic (*i.e.,* derived from an individual of the same species that is different than the patient) or xenogeneic (*i.e.,* derived from a different species than the patient). Therapeutic cells may be homogenous (*i.e.,* consisting of a single cell type) or heterogenous (*i.e.,* consisting of multiple cell types). The term "therapeutic cell" includes both therapeutically active cells as well as progenitor cells capable of differentiating into a therapeutically active cell.

A "growth culture medium" refers to a solid, liquid or semi-solid designed to support the growth of microorganisms or cells. A growth culture medium contains at least the minimum nutrients possible for colony or cell growth such as a carbon source (which may be a sugar such as glucose, or a less energy-rich source such as succinate), various salts (which may provide essential elements such as magnesium, nitrogen, phosphorus, and sulfur), and water

As used herein, "physiologically balanced" salt solution refers to a solution or medium where the concentrations of salts and other components are adjusted such that the solution or medium is isotonic with human cells, with osmolarity approximately 280 to 310 mOsmol/L, and is at a physiological pH, approximately pH 7.3-7.4. Examples of physiologically balanced salt solutions include, but are not limited to, Hank's basic salt solution, Alpha Minimum Essential Medium (aMEM), Dulbecco's Minimum Essential Medium (DMEM), Iscove's Modified Dulbecco's Medium (IMDM) and Plasma-Lyte solutions such as Plasma-Lyte A.

As used herein, "hypertonic," "isotonic," and "hypotonic" are relative terms *e.g.,* in relation to physiological osmolality regarding an osmotic differential or gradient between two compartments (such as the blood plasma and the intracellular fluid (ICF)). Accordingly, an "isotonic" solution refers any physiologically and/or pharmaceutically acceptable solution that is isotonic with respect to physiological osmolality.

To determine whether a pharmaceutical preparation is isotonic, hypertonic, or hypotonic with respect to blood, one calculates the osmolarity for all chemical components of a solution including the diluent. Tonicity can be calculated for fluids and dissolved or diluted medications, which are expressed in a numerical value of milliosmoles per liter of fluid (mOsm/L) or per kilogram of solvent (mOsm/kg). These two values also known as osmolarity and osmolality, respectively. The osmolarity of blood ranges between 285 and 310 mOsm/L and the osmolality of blood ranges between 275 and 299 mOsm/kg.

Solution osmolarity is based in part on the concepts of osmosis and osmotic pressure. Osmosis is the diffusion of solutes (dissolved particles) or the transfer of fluid through semipermeable membranes such as blood vessels or cell membranes. Osmotic pressure, which facilitates the transport of molecules across membranes, is expressed in osmolar concentrations and is referred to as hypo-osmotic (hypotonic), iso-osmotic (isotonic), or hyper-osmotic (hypertonic) when compared with biologic fluids such as blood or plasma. The term "tonicity" and "osmotic pressure" are often considered synonymous.

The osmotic pressure is the hydrostatic (or hydraulic) pressure required to oppose the movement of water through a semipermeable membrane in response to an 'osmotic gradient' (*i.e.,* differing particle concentrations on the two sides of the membrane). Serum osmolality can be measured by use of an osmometer or it can be calculated as the sum of the concentrations of the solutes present in the solution.

As used herein, tonicity and osmotic pressure are to be considered synonymously, and are to be understood broadly. Tonicity can mean the effective osmolality and is equal to the sum of the concentrations of the solutes in a solution that have the capacity to exert an osmotic force across a membrane, including a cell membrane. In the strict sense, osmolality is a property of a particular solution and is independent of any membrane. Tonicity is a property of a solution in reference to a particular membrane. However, the invention shall refer to solutions being isotonic, hypertonic, or hypotonic with respect to biological solutions such as blood or plasma, and this referencing shall include the meaning that the particular solution is isotonic hypertonic, or hypotonic with blood or plasma with respect to a cell membrane of a cell in the blood or plasma or other biological solution.

An operational definition of tonicity can be used to explain the term. This can be based on an experiment of adding a test solution to whole blood and observing the result. If the RBCs in whole blood swell and rupture, the test solution is said to be hypotonic compared to normal plasma. If the RBCs shrink and become crenate, the test solution is said to be hypertonic compared to normal plasma. If the RBCs stay the same, the test solution is said to be isotonic with plasma. The RBC cell membrane can be the reference membrane. For example, whole blood placed in normal saline (*i.e.,* 0.9% sodium chloride) will not swell, and hence normal saline is said to be isotonic.

The terms "proliferation" and "expansion" as used interchangeably herein with reference to cells, refer to an increase in the number of cells of the same type by division. The term "differentiation" refers to a developmental process whereby cells become specialized for a particular function, for example, where cells acquire one or more morphological characteristics and/or functions different from that of the initial cell type. Methods of cord blood stem cell expansion are known in the art. Such expansion techniques include those described in U.S. Pat. No. 7,399,633; WO/2013/086436, WO/2013/179633, US20180353541; Delaney et al., 2010, Nature Med. 16(2): 232-236; Zhang et al., 2008, Blood 111:3415-3423; and Himburg et al., 2010, Nature Med. 16, 475-482.

The term "differentiation" includes both lineage commitment and terminal differentiation processes. Differentiation may be assessed, for example, by monitoring the presence or absence of lineage markers, using immunohistochemistry or other procedures known to a worker skilled in the art. Differentiated progeny cells derived from progenitor cells may be, but are not necessarily, related to the same germ layer or tissue as the source tissue of the stem cells. For example, neural progenitor cells and muscle progenitor cells can differentiate into hematopoietic cell lineages.

The terms "lineage commitment" and "specification," as used interchangeably herein, refer to the process a stem cell undergoes in which the stem cell gives rise to a progenitor cell committed to forming a particular limited range of differentiated cell types. Committed progenitor cells are often capable of self-renewal or cell division.

The term "terminal differentiation" refers to the final differentiation of a cell into a mature, fully differentiated cell. For example, hematopoietic progenitor cells and muscle progenitor cells can differentiate into neural or glial cell lineages, terminal differentiation of which leads to mature neurons or glial cells. Usually, terminal differentiation is associated with withdrawal from the cell cycle and cessation of proliferation.

The term "progenitor cell," as used herein, refers to a cell that is committed to a particular cell lineage and which gives rise to cells of this lineage by a series of cell divisions. Examples of progenitor cells include precursor cells for the neuronal, hepatic, nephrogenic, adipogenic, osteoblastic, osteoclastic, alveolar, cardiac, intestinal, or endothelial lineage.

The term "culturing" refers to maintaining stem cells under conditions in which they can proliferate and avoid senescence. For example, in the present disclosure stem cells are cultured in media containing a lithium salt and optionally one or more growth factors, *i.e.,* a growth factor cocktail.

The term "umbilical cord blood" refers to a source of pluripotent and multipotent stem cells obtained from the blood of umbilical cords that are left over after birth. Examples of stem cells found in umbilical cord blood include, but are not limited to, mesenchymal stem cells, hematopoietic stem cells, and progenitor cells. Mesenchymal stem cells and progenitor cells can typically differentiate into nerve cells, marrow stromal cells, chondrocytes, osteoblasts, adipocytes, myocytes, tenocytes, and ligament cells. Hematopoietic stem cells can typically give rise to cells of the lymphoid, myeloid, and erythroid lineages. A detailed description of methods for collecting and processing cord blood is provided below.

The term "umbilical cord blood unit" refers to a volume of cord blood that is collected from a single donor. A single umbilical cord blood unit is typically used in the methods of the present invention, but multiple cord blood units, e.g., double cord blood units, can also be used to increase stem cell number.

The term "cord blood stem cells" refers to a population enriched in hematopoietic stem cells, or enriched in hematopoietic stem and progenitor cells, derived from human umbilical cord blood and/or human placental blood collected at birth. The hematopoietic stem cells, or hematopoietic stem and progenitor cells, can be positive for a specific marker expressed in increased levels on hematopoietic stem cells or hematopoietic stem and progenitor cells, relative to other types of hematopoietic cells. For example, such markers can be CD34, CD43, CD45RO, CD45RA, CD59, CD90, CD109, CD117, CD133, CD166, HLA DR, or a combination thereof. In addition, the hematopoietic stem cells, or hematopoietic stem and progenitor cells, can be negative for an expressed marker, relative to other types of hematopoietic cells. For example, such markers can be Lin, CD38, or a combination thereof. In particular embodiments, the hematopoietic stem cells, or hematopoietic stem and progenitor cells, are CD34+ cells.

As used herein, the terms "plasma is substantially depleted" and "plasma-depleted" refer to processed umbilical cord blood units in which a volume of plasma greater than about 30%, 35%, 40%, 45%, 50%, 55%, 60%, 65%, 70%, 75%, 80%, 85%, 90%, or 95% has been removed. For example, plasma can be substantially depleted by centrifuging cord blood and separating the cellular fraction from the plasma fraction. The plasma volume remaining following substantial depletion is typically from about 0% to about 30% by volume, preferably from about 10% to about 30% by volume.

The terms "non-red blood cell-depleted" and "red blood cells are not depleted" as used herein refer to processed umbilical cord blood units in which a volume of red blood cells less than about 30%, 25,%, 20%, 15%, 10%, 5%, 4%, 3%, 2%, or 1% has been removed. As used herein, the terms "red blood cell is substantially depleted" and "red blood cell-depleted" refer to processed umbilical cord blood units in which a volume of red blood cells greater than about 30%, 35%, 40%, 45%, 50%, 55%, 60%, 65%, 70%, 75%, 80%, 85%, 90%, or 95% has been removed.

"Nucleated cells" refers to cells that have a nucleus, *i.e.,* an organelle that comprises chromosomal DNA. Nucleated cells include, *e.g.,* white blood cells and stem cells. "Unnucleated cells" includes, *e.g.,* adult red blood cells.

Therapeutically effective amounts of cells within formulations can be greater than 10² cells, greater than 10³ cells, greater than 10⁴ cells, greater than 10⁵ cells, greater than 10⁶ cells, greater than 10⁷ cells, greater than 10⁸ cells, greater than 10⁹ cells, greater than 10¹⁰ cells, or greater than 10¹¹. In particular embodiments, formulations can be calibrated to provide 1 million-20 million cells per kilogram when administered to a subject

In formulations disclosed herein, cells are generally in a volume of a liter or less, 500 ml or less, 250 ml or less or 100 ml or less. Hence the density of administered cells is typically greater than 10⁷ cells/ml or 10⁸ cells/ml or more (*e.g.,* 10⁹ cells/ml).

The formulations disclosed herein can be prepared for administration by, for example, injection, infusion, perfusion, or lavage. The formulations can further be formulated for bone marrow, intravenous, intradermal, intraarterial, intranodal, intralymphatic, intraperitoneal, intralesional, intraprostatic, intravaginal, intrarectal, topical, intrathecal, intratumoral, intramuscular, intravesicular, and/or subcutaneous injection.

An "effective amount" is the amount of cells necessary to result in a desired physiological change in a subject. A "prophylactic treatment" includes a treatment administered to a subject who does not display signs or symptoms of a condition such that treatment is administered for the purpose of diminishing, preventing, or decreasing the risk of developing the condition. A "therapeutic treatment" includes a treatment administered to a subject who displays symptoms or signs of a condition and is administered to the subject for the purpose of reducing the severity or progression of the condition. A therapeutic treatment can also partially or completely resolve the condition.

The term "therapeutic composition" or pharmaceutical composition" refers to the combination of an active agent with a carrier, inert or active, making the composition especially suitable for diagnostic or therapeutic use *in vivo* or *ex vivo.*

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. A "pharmaceutically acceptable carrier," after administered to or upon a subject, does not cause undesirable physiological effects. The carrier in the pharmaceutical composition must be "acceptable" also in the sense that it is compatible with the active ingredient and can be capable of stabilizing it. One or more solubilizing agents can be utilized as pharmaceutical carriers for delivery of an active compound. Examples of a pharmaceutically acceptable carrier include, but are not limited to, biocompatible vehicles, adjuvants, additives, and diluents to achieve a composition usable as a dosage form. Examples of other carriers include colloidal silicon oxide, magnesium stearate, cellulose, and sodium lauryl sulfate.

The term "subject" includes human and non-human animals. The preferred subject for treatment is a human. As used herein, the terms "subject" and "patient" are used interchangeably irrespective of whether the subject has or is currently undergoing any form of treatment. As used herein, the terms "subject" and "subjects" may refer to any vertebrate, including, but not limited to, a mammal (*e.g*., cow, pig, camel, llama, horse, goat, rabbit, sheep, hamsters, guinea pig, cat, dog, rat, and mouse, a non-human primate (for example, a monkey, such as a cynomolgous monkey, chimpanzee, etc) and a human). In one embodiment, the subject is a human. In another embodiment, the subject is an experimental, non-human animal or animal suitable as a disease model.

As used herein, "treating" or "treatment" refers to administration of a compound or agent or a composition to a subject who has a disorder or is at risk of developing the disorder with the purpose to cure, alleviate, relieve, remedy, delay the onset of, prevent, or ameliorate the disorder, the symptom of the disorder, the disease state secondary to the disorder, or the predisposition toward the disorder. The terms "prevent," "preventing," "prevention," "prophylactic treatment" and the like refer to reducing the probability of developing a disorder or condition in a subject, who does not have, but is at risk of or susceptible to developing a disorder or condition. "Ameliorating" generally refers to the reduction in the number or severity of signs or symptoms of a disease or disorder.

The term "administer" refers to a method of delivering agents, compounds, or compositions to the desired site of biological action. These methods include, but are not limited to, topical delivery, parenteral delivery, intravenous delivery, intradermal delivery, intramuscular delivery, intrathecal delivery, colonic delivery, rectal delivery, or intraperitoneal delivery.

The term "about" or "approximately" means within an acceptable range for the particular value as determined by one of ordinary skill in the art, which will depend in part on how the value is measured or determined, e.g., the limitations of the measurement system. For example, "about" can mean a range of up to 20%, preferably up to 10%, more preferably up to 5%, and more preferably still up to 1% of a given value. Unless otherwise stated, the term "about" means within an acceptable error range for the particular value.

### EXAMPLES

### Example 1

This example describes an exemplary procedure for packaging umbilical cord blood cells freshly collected or thawed from frozen STEMCYTE Umbilical Cord Blood Units (UCBU). Briefly, cord blood cells were collected using standard methods known in the art. Alternatively, one or more bags of frozen UCBCs were thawed according to the procedure described WO2012112572. The cells were then subjected either to a blood lysate procedure or to a MNC isolation procedure a described in WO2012112572. The cells were then mixed with pharmaceutically acceptable carrier/preservation solutions containing about 1% HAS. Two pharmaceutically acceptable carriers/preservation solutions used here were saline and PLASMA-LYTE A. The concentration of the cells were adjusted to about 1 x 10⁹ /ml. The cells thus packaged were transported at room temperature or 4 °C to a different site over periods of 12 hours to 96 hours (4 days).

### Example 2

In this example, assays were conducted to examine cells packaged and shipped in the manner described in Example 1 above. Briefly, the cell packages were inspected and unpacked in the manner described in WO2012112572. Cell viability assays, cell count of the UCB-MNC, and CFU assays were carried out in the manner described in WO2012112572. The results are shown in Figs. 1A-1D.

As shown in the figures, the cells packaged and shipped in PLASMA-LYTE A at 4 °C ("P-cold") showed higher viability (by acridine orange/propidium iodide (AO/PI) stain), total CFU numbers, total nucleated cells (TNC) recovery, and cells expressing CD34/CD45 markers than other conditions, such as PLASMA-LYTE A at room temperature ("P-rt"), saline at room temperature ("S-rt"), and saline at 2-8 °C ("S-cold"). For example, after about 72 hours (3 days) of shipping or storage, the cells packaged and shipped in PLASMA-LYTE A at 2-8 °C ("P-cold") exhibited greater than 80% viability, greater than 80% TNC recovery, more than 90 CFU/plate (per 3x10⁴ cells plated), and greater than 0.5% CD34⁺/CD45⁺ cells.

The foregoing examples and description of the preferred embodiments should be taken as illustrating, rather than as limiting the present invention as defined by the claims.

## Claims

1. A therapeutic composition comprising (i) about 1 x 10⁷ to 1 x 10⁹ /ml umbilical cord blood cells and (ii) a pharmaceutically acceptable carrier solution that (a) contains about 25-30 mM acetate and about 20-25 mM gluconate and (b) has an osmolarity of about 270 to 320 mOsmol/L, wherein the therapeutic composition is free of DMSO or contains a trace amount of DMSO.

2. The therapeutic composition of claim 1, wherein the pharmaceutically acceptable carrier solution contains one or more of the following: about 120-160 mM Na⁺, about 3-7 mM K⁺, about 1.0-2.0 mM Mg²⁺, and about 90-110 mM Cl⁻.

3. The therapeutic composition of claim 2, wherein the pharmaceutically acceptable carrier solution is free of Ca²⁺ or lactate or both.

4. The therapeutic composition of any one of claims 1-3, wherein the pharmaceutically acceptable carrier solution contains: about 140 mM Na⁺, about 5 mM K⁺, about 1.5 mM Mg²⁺, about 98 mM Cl⁻, about 27 mM acetate, and about 23 mM gluconate.

5. The therapeutic composition of claim 4, wherein the pharmaceutically acceptable carrier solution contains: about 90 mM Sodium Chloride (NaCl), about 5 mM Potassium Chloride (KCl), about 1.5 mM Magnesium Chloride (MgCl₂•6H₂O), about 27 mM Sodium Acetate Trihydrate (C₂H₃NaO₂•3H₂O), and about 23 mM Sodium Gluconate (C₆H₁₁NaO₇).

6. The therapeutic composition of claim 1, wherein the pharmaceutically acceptable carrier solution has 126-154 mEq/L sodium.

7. The therapeutic composition of any one of claims 1-6, wherein the pharmaceutically acceptable carrier solution has a pH of 5.5 to 8.0.

8. The therapeutic composition of any one of claims 1-7, wherein the therapeutic composition contains about 1 x 10⁸ /ml umbilical cord blood cells.

9. The therapeutic composition of any one of claims 1-8, wherein the umbilical cord blood cells are CD34⁺,cells.

10. A packaging product comprising
a composition of any one of claims 1-9, and
a container holding the composition and comprising a substrate, wherein the substrate comprises a polymer.

11. The packaging product of claim 10, wherein the container is a bag, a tube, a syringe, or a vial for an injector, and preferably the container is sealed.

12. A method for storing or shipping cells, comprising (i) providing the therapeutic composition or packaging product of any one of claims 1-11, and (ii) storing or shipping the composition for about 24 to 96 hours at a temperature within the range of 1 - 10 °C.

## Patentansprüche

1. Therapeutische Zusammensetzung, umfassend (i) etwa 1 x 10⁷ bis 1 x 10⁹ /ml Nabelschnurblutzellen und (ii) eine pharmazeutisch verträgliche Trägerlösung, die (a) etwa 25-30 mM Acetat und etwa 20-25 mM Gluconat enthält und (b) eine Osmolarität von etwa 270 bis 320 mOsmol/L aufweist, wobei die therapeutische Zusammensetzung frei von DMSO ist oder nur Spuren von DMSO enthält.

2. Die therapeutische Zusammensetzung nach Anspruch 1, wobei die pharmazeutisch verträgliche Trägerlösung eines oder mehreres des Folgenden enthält: etwa 120-160 mM Na⁺, etwa 3-7 mM K⁺, etwa 1,0-2,0 mM Mg²⁺ und etwa 90-110 mM Cl⁻.

3. Die therapeutische Zusammensetzung nach Anspruch 2, wobei die pharmazeutisch verträgliche Trägerlösung frei von Ca²⁺ oder Laktat oder von beidem ist.

4. Die therapeutische Zusammensetzung nach einem der Ansprüche 1 bis 3, wobei die pharmazeutisch verträgliche Trägerlösung Folgendes enthält: etwa 140 mM Na⁺, etwa 5 mM K⁺, etwa 1,5 mM Mg2+, etwa 98 mM Cl⁻, etwa 27 mM Acetat und etwa 23 mM Gluconat.

5. Die therapeutische Zusammensetzung nach Anspruch 4, wobei die pharmazeutisch verträgliche Trägerlösung Folgendes enthält: etwa 90 mM Natriumchlorid (NaCl), etwa 5 mM Kaliumchlorid (KCl), etwa 1,5 mM Magnesiumchlorid (MgCl₂•6H₂O), etwa 27 mM Natriumacetat-Trihydrat (C₂H₃NaO₂•3H₂O) und etwa 23 mM Natriumgluconat (C₆H₁₁NaO₇)

6. Die therapeutische Zusammensetzung nach Anspruch 1, wobei die pharmazeutisch verträgliche Trägerlösung 126-154 mEq/L Natrium enthält.

7. Die therapeutische Zusammensetzung nach einem der Ansprüche 1 bis 6, wobei die pharmazeutisch verträgliche Trägerlösung einen pH-Wert von 5,5 bis 8,0 aufweist.

8. Die therapeutische Zusammensetzung nach einem der Ansprüche 1 bis 7, wobei die therapeutische Zusammensetzung etwa 1 x 10⁸ /ml Nabelschnurblutzellen enthält

9. Die therapeutische Zusammensetzung nach einem der Ansprüche 1-8, wobei es sich bei den Nabelschnurblutzellen um CD34⁺-Zellen handelt

10. Verpackungsprodukt, umfassend
eine Zusammensetzung nach einem der Ansprüche 1 bis 9 und
einen Behälter, der die Zusammensetzung enthält und ein Substrat umfasst, wobei das Substrat ein Polymer umfasst

11. Das Verpackungsprodukt nach Anspruch 10, wobei es sich bei dem Behälter um einen Beutel, ein Röhrchen, eine Spritze oder ein Fläschchen für einen Injektor handelt und der Behälter vorzugsweise versiegelt ist

12. Verfahren zum Lagern oder Versenden von Zellen, umfassend (i) Bereitstellen der therapeutischen Zusammensetzung oder des Verpackungsprodukts nach einem der Ansprüche 1-11 und (ii) Lagern oder Versenden der Zusammensetzung für etwa 24 bis 96 Stunden bei einer Temperatur im Bereich von 1 bis 10 °C

## Revendications

1. Une composition thérapeutique comprenant (i) environ 1 x 10⁷ à 1 x 10⁹ cellules de sang de cordon ombilical par mL et (ii) une solution de véhicule pharmaceutiquement acceptable qui (a) contient environ 25- 30 mM d'acétate et environ 20- 25 mM de gluconate et (b) a une osmolarité d'environ 270 à 320 mOsmol/L, dans laquelle la composition thérapeutique est exempte de DMSO ou contient des quantités en traces de DMSO.

2. La composition thérapeutique selon la revendication 1, dans laquelle la solution de véhicule pharmaceutiquement acceptable contient un ou plusieurs des suivants : environ 120- 160 mM de Na⁺, environ 3-7 mM de K⁺, environ 1,0 -2,0 mM de Mg²⁺, et environ 90 110 mM de Cl⁻ .

3. La composition thérapeutique selon la revendication 2, dans laquelle la solution de véhicule pharmaceutiquement acceptable est exempte de Ca²⁺ ou de lactate ou des deux.

4. La composition thérapeutique selon l'une quelconque des revendications 1-3, dans laquelle la solution de véhicule pharmaceutiquement acceptable contient : environ 140 mM de Na⁺, environ 5 mM de K⁺, environ 1,5 mM de Mg²⁺, environ 98 mM de Cl⁻ ,environ 27 mM d'acétate à, et environ 23 mM de gluconate.

5. La composition thérapeutique selon la revendication 4, dans laquelle la solution de véhicule pharmaceutiquement acceptable contient : du environ 90 mM de chlorure de sodium (NaCl), environ 5 mM de chlorure de potassium (KCl), environ 1,5 mM de chlorure de magnésium (MgCl₂•6H₂O), environ 27 mM d''acétate de sodium trihydraté (C₂H₃NaO₂•3H₂O), et environ 23 mM de gluconate de sodium (C₆H₁₁NaO₇).

6. La composition thérapeutique selon la revendication 1, dans laquelle la solution de véhicule pharmaceutiquement acceptable a 126- 154 mEq/l de sodium.

7. La composition thérapeutique selon l'une quelconque des revendications 1- 6, dans laquelle la solution de véhicule pharmaceutiquement acceptable a un pH de 5,5 à 8,0.

8. La composition thérapeutique selon l'une quelconque des revendications 1- 7, dans laquelle la composition thérapeutique contient environ 1 x 10⁸ cellules de sang de cordon ombilical par mL.

9. La composition thérapeutique selon l'une quelconque des revendications 1- 8, dans laquelle les cellules de sang de cordon ombilical sont des cellules, CD34⁺.

10. Un produit de conditionnement comprenant
une composition de l'une quelconque des revendications 1- 9, et
un récipient contenant la composition et comprenant un substrat, dans lequel le substrat comprend un polymère.

11. Le produit de conditionnement selon la revendication 10, dans lequel le récipient est un sac, un tube, une seringue, ou un flacon pour un injecteur, et de préférence le récipient est scellé.

12. Une méthode pour stocker ou expédier des cellules, comprenant (i) la fourniture de la composition thérapeutique ou du produit de conditionnement de l'une quelconque des revendications 1-11, et (ii) le stockage ou l'expédition de la composition pendant environ 24 à 96 heures à une température située dans la plage de 1- 10 °C.
